# EUROPEAN PATENT APPLICATION

(11) **EP 1 714 616 A1**
(43) Date of publication of application: **25.10.2006**
(21) Application number: 06004210.8
(22) Date of filing: 02.03.2006
(51) Int. Cl.: A61B 17/00

(54) **Specimen retrieval apparatus**

(30) Priority: 07.03.2005 US 73551
(71) Applicant: Tyco Healthcare Group LP, Norwalk, Connecticut 06856 (US)
(72) Inventor: Aranyi, Ernest, Easton, CT 06612 (US)
(74) Representative: HOFFMANN EITLE

(57) **Abstract**

A specimen removal apparatus includes a pouch assembly (290) fabricated from a flexible membrane, a pouch support (230), a drawstring thread (250) forming a running noose disposed circumferentially round the end of the pouch (260), an endoscopic tubular portion (180), and a pusher rod (190) having an aperture (194) for permitting the passage therethrough of a single thread. When the drawstring thread (250) is pulled, the knot (251) is stopped at the aperture (194) and the noose is closed, thereby closing the mouth (262) of the pouch (260). The pouch assembly (290) is detachable from the apparatus.

## Description

### BACKGROUND

### 1. Field of the Invention

The present disclosure relates to a surgical containment apparatus and methods for use thereof. More particularly, the present disclosure relates to a specimen retrieval pouch and method for use in minimally invasive surgical procedures.

### 2. Background of the Art

Laparoscopic and endoscopic surgical procedures are minimally invasive procedures in which operations are carried out within the body by using elongated instruments inserted through small entrance openings in the body. The initial opening in the body tissue to allow passage of the endoscopic or laparoscopic instruments to the interior of the body may be a natural passageway of the body, or it can be created by a tissue piercing instrument such as a trocar. Laparoscopic and endoscopic procedures generally require that any instrumentation inserted in the body be sealed, i.e. provisions must be made to ensure that gases do not enter or exit the body through the instrument or the entrance incision so that the surgical region of the body, e.g. the peritoneum, may be insufflated. Mechanical actuation of such instruments is for the most part constrained to the movement of the various components along a longitudinal axis with structure provided to convert longitudinal movement to lateral movement where necessary.

Because the endoscopic or laparoscopic tubes, instrumentation, and any required punctures or incisions are relatively narrow, endoscopic or laparoscopic surgery is less invasive as compared to conventional surgical procedures in which the surgeon is required to cut open large areas of body tissue. Therefore, laparoscopic or endoscopic surgery minimizes trauma to the patient and reduces patient recovery time.

Minimally invasive procedures may be used for partial or total removal of body tissue or organs from the interior of the body, e.g. nephrectomy, cholecystectomy, and other such procedures. During such procedures, it is common that a cyst, tumor, or other affected tissue or organ must be removed via the access opening in the skin, or through a cannula. Various types of entrapment devices have been disclosed to facilitate this procedure.

For example, U.S. Pat. No. 5,037,379 to Clayman et al. discloses a surgical tissue bag for percutaneously debulking tissue by morcellation. The bag includes a layer of puncture-resistant material, a layer of moisture-resistant material and a drawstring. In a disclosed method of use, the bag is placed within the body cavity, the body tissue or organ is placed within the bag, the opening of the bag is pulled through the incision in the skin leaving the distal end of the bag containing the tissue or organ within the body cavity, a morcellator is then inserted into the bag, and then the tissue or organ is debulked and suctioned out of the bag.

U.S. Pat. No. 5,074,867 to Wilk discloses a planar membrane having filaments attached to its corners. The membrane is placed within a body cavity with the filaments extending through the trocar cannula to the outside of the body. The organ or tissue to be removed is placed on the membrane and the filaments are pulled to close the membrane around the organ and draw it through the cannula, if the organ is sufficiently deformable. If the organ is not sufficiently deformable, e.g. because of the presence of gallstones, a forceps or other instrument is used to crush the stones or tissue.

Improvements to prior art entrapment devices are disclosed in U.S. Patent No. 5,647,372 to Tovey et al. and in U.S. Patent No. 5,465,731 to Bell et al. These disclosures are hereby incorporated by reference in their entirety.

### SUMMARY

The present disclosure is directed towards an apparatus for removing body tissue from the interior of the body as part of a minimally invasive surgical procedure. The apparatus includes a pouch assembly and a pouch support. The pouch assembly includes a number of sacs where each sac has a different diameter forming a staggered or stepped arrangement of the sacs. Each sac has a mouth at one end. One of the sacs, preferably the most distal sac, has a closed end thereby forming a cavity therein, while the other sacs have an orifice opposite the mouth of the sac. The pouch assembly may have a scored line on one of the sacs to facilitate detachment of the pouch assembly from the support. The pouch assembly can have a scored line extending circumferentially therearound between the locations of the spring structure and the drawstring thread. The pouch support can be attached to the drive structure. The detachment can be simultaneous with the closing of the pouch assembly in response to pulling the drawstring thread.

The apparatus may further include a drawstring thread forming a running noose disposed circumferentially around the pouch assembly in proximity to the openable end thereof; attachment structure for slidably attaching a first end portion of the drawstring thread to a second end portion of the drawstring thread to from the running noose; an endoscopic tubular portion having a distal end for insertion into a body; drive structure for moving the pouch (i.e., pushing or pulling the pouch) through the endoscopic tubular portion; and stop structure having an aperture for permitting passage therethrough of a single thread, the second end portion of the drawstring thread extending through the aperture, and the aperture possessing a surface for abutting and holding the attachment structure.

The sacs can be fabricated from a material selected from the group consisting of polyurethane and latex and preferably is transparent. One or more reinforced regions or bands extend circumferentially about the pouch assembly and overlap the junction between a pair of adjacent sacs. A running knot is the preferred attachment structure.

Stop structure is provided by a distal surface of the pusher structure. The pusher structure can be an elongated rod slidably disposed within the tubular portion. In the embodiment described below having only a single drawstring, thread the aperture of the stop structure has a diameter of large enough dimension to permit passage therethrough of only a single filament, but smaller dimension than the attachment structure. The aperture can be oriented parallel to the longitudinal axis of the pusher rod or transverse to the longitudinal axis of the pusher rod.

The apparatus can further include structure for resiliently opening the openable end of the pouch assembly, such as spring structure circumferentially attached to the openable end of the pouch assembly and movable between an elongated and narrow closed configuration and a rounded open configuration, the spring structure being resiliently biased to the open configuration. The spring structure, which can support the pouch assembly as well as open it, is attached to the distal end of the drive structure and is slidably movable through the tubular portion when in the closed configuration, and resiliently moveable to its open configuration when moved outside said tubular portion. The spring structure can include two elastic prongs each having a proximal end portion having a side surface in facing relation to the side surface of the proximal end portion of the other elastic prong and fastened thereto, and each elastic prong further having a distal end portion joined to the distal end portion of the other prong by a flexible membrane, such as shrink-wrap type tubing, attached to both said end portions.

The apparatus preferably further includes at least one gaseous sealing structure, such as a coating of viscous sealing material applied to the outer surfaces of the pusher structure and the drawstring thread. A knife or other cutting structure may be provided to cut the drawstring thread.

A further aspect of the present disclosure is a method of retrieving tissue. The apparatus is inserted through a cannula which has been inserted into a body. The pouch assembly is deployed by advancing the drive structure. The body tissue is severed, if necessary and placed within the pouch assembly. The pouch assembly is then closed and detached from the apparatus. The neck of the pouch assembly can then be brought to the distal end of the trocar and the whole assembly removed. Alternatively, the pouch assembly containing the specimen of body tissue may be "parked" by permitting it to remain in the body cavity until a later time during the operation whereupon the pouch assembly may be removed in conjunction with the same cannula, an alternative cannula, or through an opening in the wall of body tissue. Also contemplated is the debulking of the body tissue specimen by, for example, morcellation, or cutting, in order to facilitate its removal through a cannula.

### BRIEF DESCRIPTION OF THE DRAWINGS

Preferred embodiments of the present disclosure are described hereinbelow with reference to the drawings wherein:
FIG. 1 is a side cross-sectional view of a prior art specimen bag including a specimen partially removed from a body cavity;
FIG. 2 is a side cross-sectional view of a specimen bag according to the present disclosure including a specimen partially removed from a body cavity;
FIG. 3 is an elevational partially cut away view of the specimen removal pouch assembly according to an embodiment of the present disclosure;
FIG. 3A is another embodiment of the specimen removal pouch assembly of FIG. 3 having reinforced regions;
FIG. 3B is an alternate embodiment of the specimen removal pouch of FIG. 3A;
FIG. 4 is a perspective view of the apparatus of the present disclosure in the deployed configuration;
FIG. 5 is a perspective view of the apparatus in the initial, undeployed configuration;
FIG. 6 is an exploded perspective view of the apparatus;
FIGS. 6a and 6b illustrate, respectively, in perspective view a running knot, and, as an alternative, an eyelet structure for slidably attaching a first end portion of the drawstring thread to a second end portion for form a running loop.
FIGS. 7a, 7b, 8 and 9 are, respectively, plan, elevational, perspective, and end views of the drive rod;
FIG. 8a illustrates a side view of O-ring 210a;
FIG. 10 is a bottom plan view of the finger loop;
FIGS. 10a and 10b are perspective views of alternative finger loops;
FIG. 10c is a perspective view of a pull ring for engagement with the alternative finger loop;
FIG. 11 is a detached view of the fixture at the proximal end of the finger loop;
FIG. 12 illustrates the insertion of the apparatus of the present disclosure through a trocar cannula into a body cavity;
FIGS. 13 and 14 illustrate deployment of the specimen removal pouch;
FIG. 15 illustrates the entrapment of a tissue specimen;
FIGS. 16, 17, and 18 illustrate closure of the pouch;
FIG. 19 illustrates cutting of the drawstring;
FIG. 20 illustrates removal of the apparatus from the cannula; and
FIGS. 21 and 22 illustrate follow-up procedures for removal of the pouch.

### DETAILED DESCRIPTION OF PREFERRED EMBODIMENT

As used herein with reference to the present disclosure, the terms "laparoscopic" and "endoscopic" are interchangeable and refer to instruments having a relatively narrow operating portion for insertion into a cannula or a small incision in the skin, or to a surgical procedure in which such instruments are employed. Use herein of the term "laparoscopic" should not be construed so as to exclude "endoscopic" and use herein of the term "endoscopic" should not be construed so as to exclude "laparoscopic." To the contrary, it is believed that the present disclosure may find use in any procedure where access to the interior of the body is limited to a relatively small region of access, with or without the use of a cannula, including, but not limited to, laparoscopic procedures.

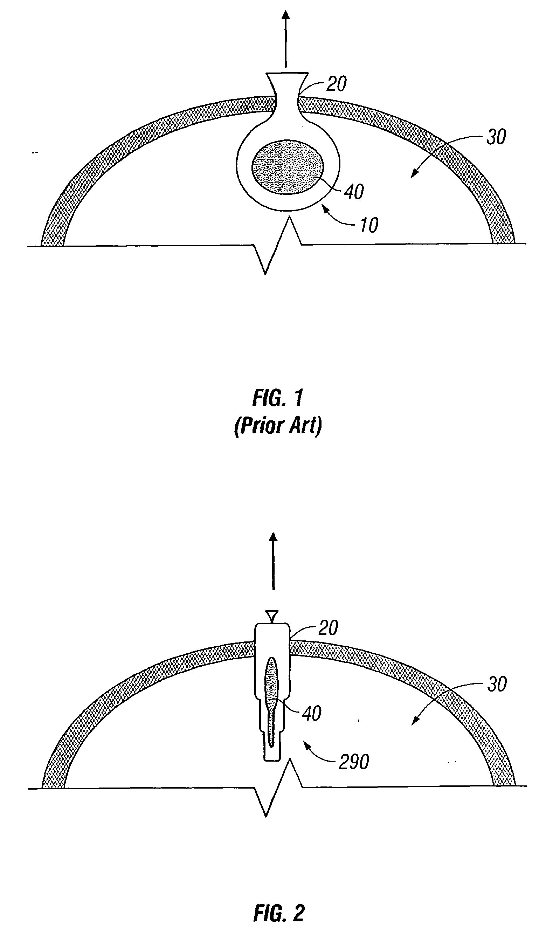

An example of a prior art entrapment device is illustrated in FIG. 1. As shown in FIG. 1, a prior art pouch 10 contains a captured tissue specimen 40 and the pouch 10 is being removed from the body cavity 30 through an opening 20 in a direction shown by the arrow. Depending on the size of the tissue specimen 40 being retrieved and the geometry of the pouch 10, it is possible for the tissue specimen 40 to agglomerate irregularly or "bunch up" inside the pouch 10 thereby inhibiting retrieval of the tissue specimen 40 through the opening 20. FIG. 2 illustrates the retrieval of the tissue specimen 40 using a pouch assembly 290 according to an embodiment of the present disclosure. The pouch assembly 290 includes a number of sacs of differing diameters operatively connected to one another that is discussed in further detail hereinbelow. Using the same size tissue specimen 40 as shown in FIG. 1, retrieving the pouch assembly 290 through the opening 20 minimizes the agglomeration or "bunching up" of the tissue specimen 40 as the pouch assembly 290 is retrieved from the body cavity 30.

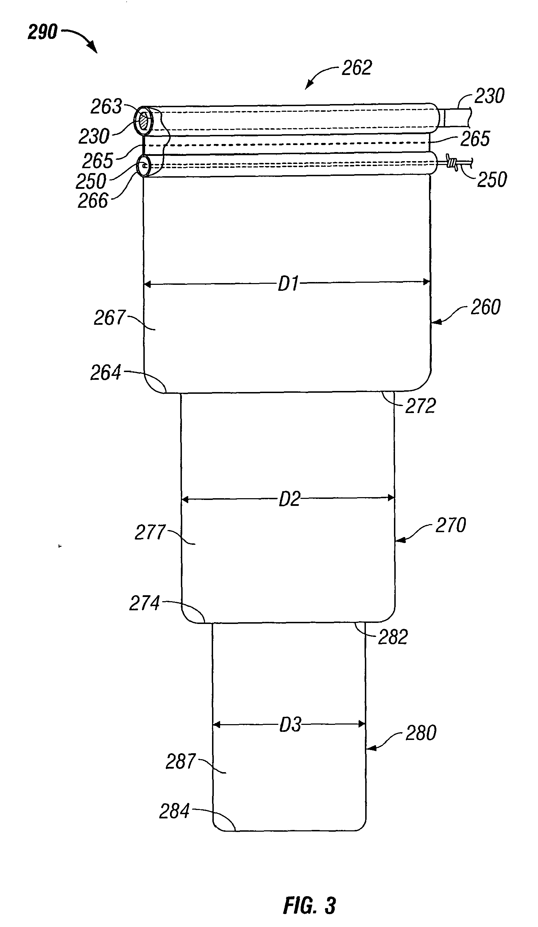

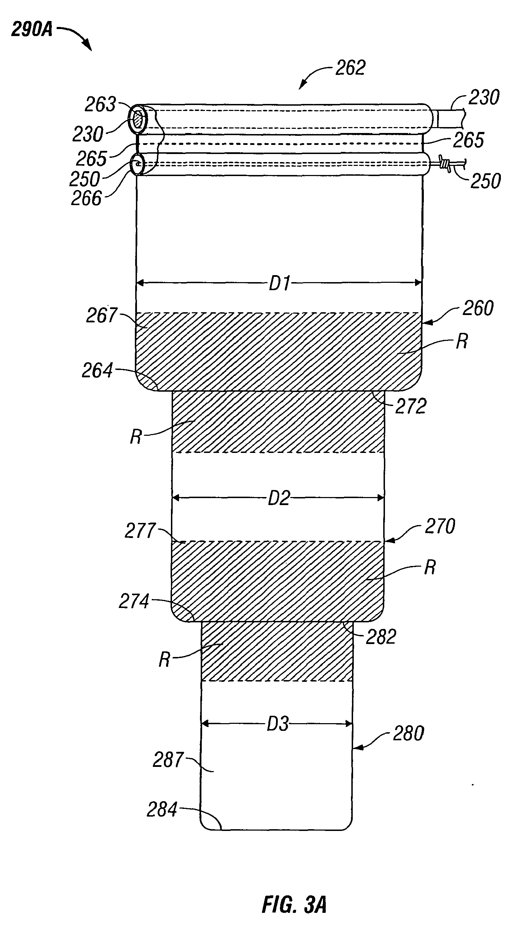

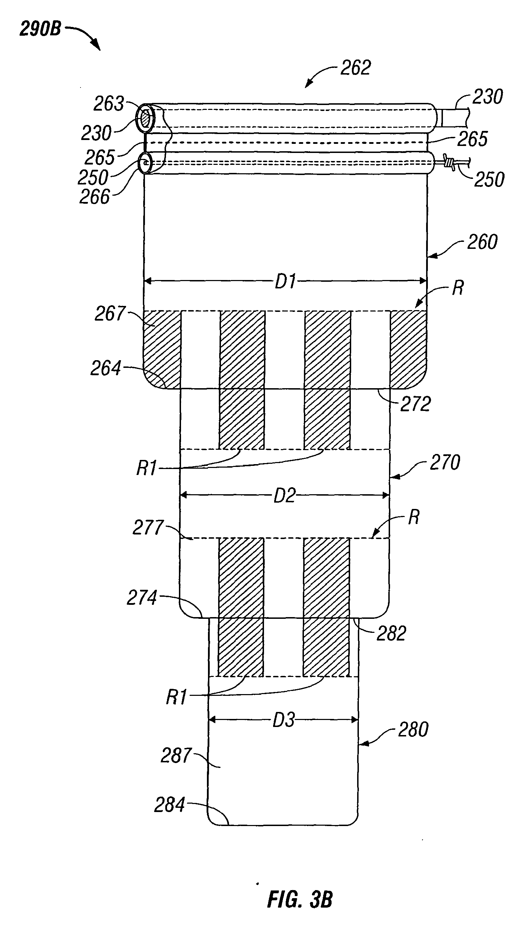

Referring specifically to FIG. 3, the removal pouch or pouch assembly 290 includes a first sac 260, a second sac 270, and a third sac 280. Each sac is formed from a flexible film or sheet that is preferably a substantially transparent polymeric material. One preferred material is polyurethane sheet, although other biocompatible materials capable of forming a flexible membrane, such as latex, may be used. It is also preferred that the material selected be between about 0.001 to about 0.005 inches in thickness, although other ranges of thickness may be used as appropriate. Preferably, the material is transparent to permit viewing the contents of the pouch assembly 290. In a preferred configuration, each sac is formed from an aromatic polyester type thermoplastic polyurethane such as Dureflex®, a product of Deerfield Urethane, Inc. in Whately, Massachusetts. The sac material is desirably impervious to liquids and/or gases. In addition, the sac material should be impervious to penetration by cancer cells.

The pouch assembly 290 may be of any dimensions suitable for the purpose of organ entrapment or removal. In the present embodiment, the first sac 260 has a diameter D1 in the range of about 1.5 inches to about 6.0 inches. Overall, the pouch assembly 290 has a depth of from about 2 inches to about 10 inches, and has a cubic capacity of up to about 2.0 liters of water, depending upon the dimensions of the pouch assembly 290.

The first sac 260 includes a mouth 262 and an orifice 264 at opposing ends defining a throat 267 therebetween. Preferably, the diameter of the throat 267 is equal to diameter D1 and is substantially uniform from the mouth 262 to the orifice 264. Alternately, the first sac 260 may be tapered from the mouth 262 to the orifice 264 forming a frustoconical or an inverted frustoconical shape. In addition, other shapes and configurations of the sac are contemplated. The mouth 262 has both an open and a closed configuration, while the orifice 264 only has an open configuration.

In particular, the first sac 260 possesses a linear portion weakened by perforation or, more preferably, scoring, which extends circumferentially around the mouth 262 of the first sac 260 between proximal and distal sleeves 263 and 266, respectively. A scored line 265 may be created by induction heating to create a linear portion having thickness less than that of the original material to facilitate tearing of the material along the scored line 265.

The proximal sleeve 263 is adapted to receive a spring member 230, described below. The distal sleeve 266 is adapted to receive a drawstring 250. The scored line 265 is adapted to tear when the drawstring 250 is pulled with sufficient force to close the mouth 262 of the first sac 260 distal to the scored line 265, thereby providing fast detachment of pouch assembly 290 from the spring member 230 simultaneously with closure of mouth 262. Clearly, alternative structures also can be utilized to detach the pouch assembly 290 from the spring member 230, such as by pulling with a grasper or by cutting with a scissors.

Still referring to FIG. 3, the second sac 270 has a mouth 272 and an orifice 274 at opposing ends defining a throat 277 therebetween. Similar to the first sac 260, the second sac 270 has a diameter D2 that is substantially uniform from the mouth 272 to the orifice 274. The mouth 272 is open and in communication with the throat 267 of the first sac 260. It is preferred that the diameter D2 or the diameter of the mouth 272 is less than the diameter D1 or the diameter of the orifice 264. Configured thusly, the throats 267, 277 of the first and second sacs 260, 270, respectively, are in fluid communication with each other and form a staggered or stepped arrangement of the sacs included in the pouch assembly 290. Alternately, the second sac 270 may be tapered from the mouth 272 to the orifice 274 forming a frustoconical or an inverted frustoconical shape. In addition, other shapes and configurations of the sac are contemplated.

The third sac 280 includes a mouth 282 and a base 284 at opposing ends. The mouth 282 is open while the base 284 is closed defining a cavity 287 therein. The cavity has a diameter D3 that is preferably uniform throughout. The mouth 282 is in fluid communication with the throat 277 of the second sac 270. It is preferred that the diameter D3 or the diameter of the mouth 282 is less than the diameter D2 or the diameter of the orifice 274. In this configuration, the throats 267, 277, and 287 are in fluid communication with one another to form a staggered arrangement of the pouch assembly 290. Alternately, the third sac 280 may be tapered from the mouth 282 to the base 28 forming a frustoconical or inverted frustoconical shape. In addition, other shapes and configurations of the sacs are contemplated.

Referring now to FIG. 3A, an alternate embodiment of the pouch assembly 290 is disclosed. The pouch assembly 290A according to this embodiment includes the same or similar components as the embodiment shown in FIG. 3a and further includes a reinforced band or region R. The reinforced region R overlaps the junction between an orifice and a mouth of a pair of adjacent sacs (e.g. orifice 264 of sac 260 and mouth 272 of sac 270). The dimensions (i.e. thickness and/or height) of the reinforced region R may be influenced by a number of factors including, but not limited to, dimensions of the pouch assembly 290A, dimensions of the adjoining sacs, and the task being performed. The reinforced region R improves the overall rigidity of the pouch assembly 290A and helps maintain the staggered or stepped shape of the pouch assembly 290A. Additionally, the reinforced regions R improve the strength of the joint between the adjacent sacs thereby minimizing the possibility that the sacs will separate during a surgical procedure or increasing the size and/or mass of the tissue sample to be collected. In preferred embodiments, the reinforced region R extends circumferentially about the pouch assembly 290A although it is contemplated that it may only extend for a portion of a circumference of the pouch assembly 290A.

Alternatively, as illustrated in FIG. 3B, another embodiment of a pouch assembly 290B includes a reinforced region R having a plurality of reinforced sections R1 that are circumferentially spaced apart forming gaps therebetween. It is preferred that each reinforced section R1 have substantially identical dimensions (i.e. thickness, height, and width), although it is contemplated that the dimensions of the reinforced sections R1 may be varied for the same or similar reasons discussed for the embodiment of FIG. 3A. As in the previous embodiment, the reinforced region R may extend circumferentially about the pouch assembly 290B or only for a portion thereof. In either of the embodiments of FIGS. 3A or 3B, the reinforced region R may be included in some or all of the pairs of adjacent sacs.

Alternatively, the pouch assembly may only include two sacs where the second sac has a closed end opposite its mouth defining a cavity therein. In other embodiments of the disclosure, additional sacs may be included, such as more than three sacs, with the last or most distal sac having a closed end opposite its mouth to define a cavity therein. These alternative configurations increase the flexibility and utility of the pouch assembly 290 of the present disclosure. The pouch assembly may be formed from discrete sacs where the sacs are bonded or joined together using known methods such that each bond is substantially fluid-tight. Alternatively, the pouch assembly may be monolithically formed using known methods to create the staggered arrangement of the included sacs. The pouch assembly of these alternate embodiments may include reinforced bands or regions as previously discussed.

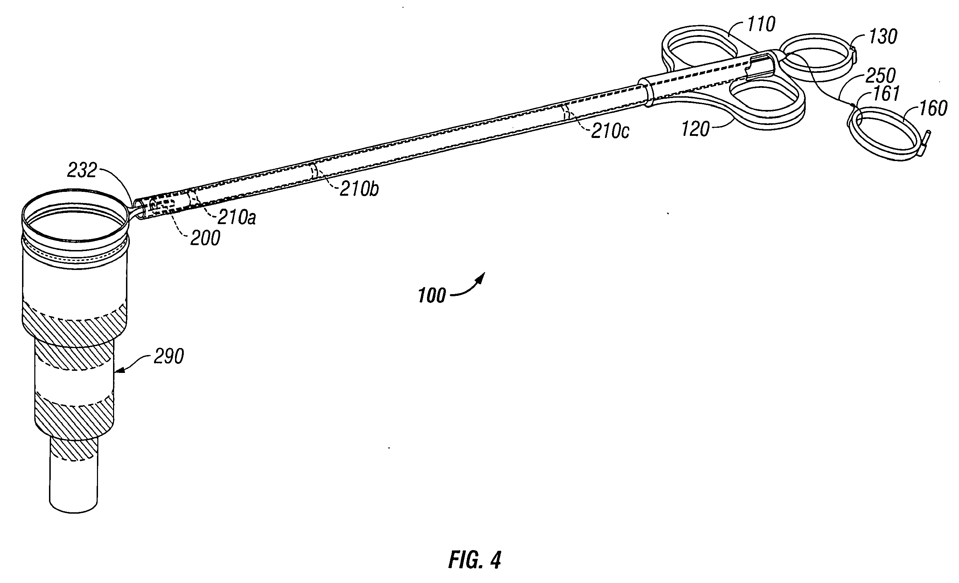

Referring now to FIGS. 4, 5, and 6, the laparoscopic removal pouch and applicator assembly includes the elongated tube 180 which is of such dimensions to be insertable through a trocar cannula for endoscopic or laparoscopic procedures.

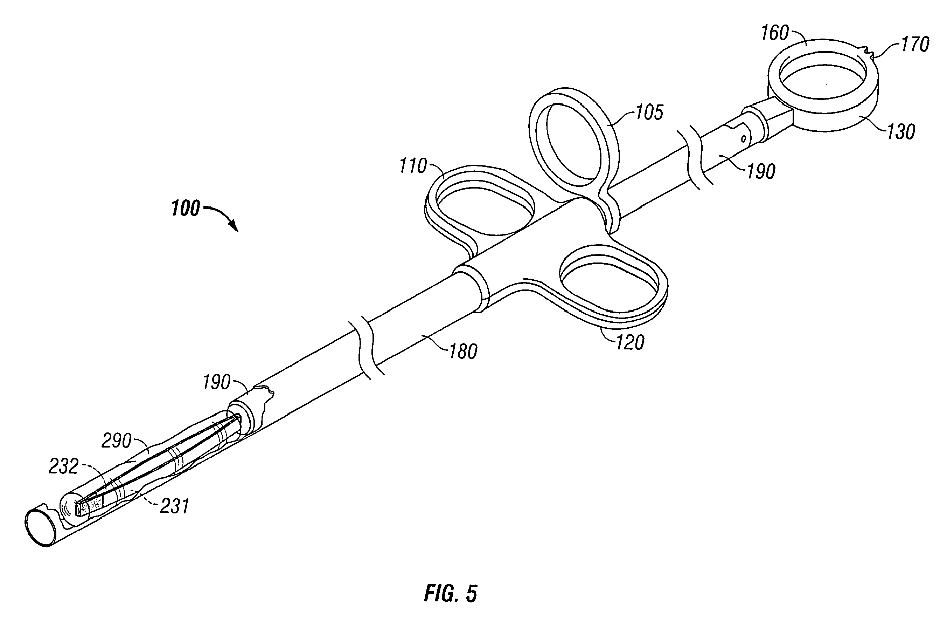

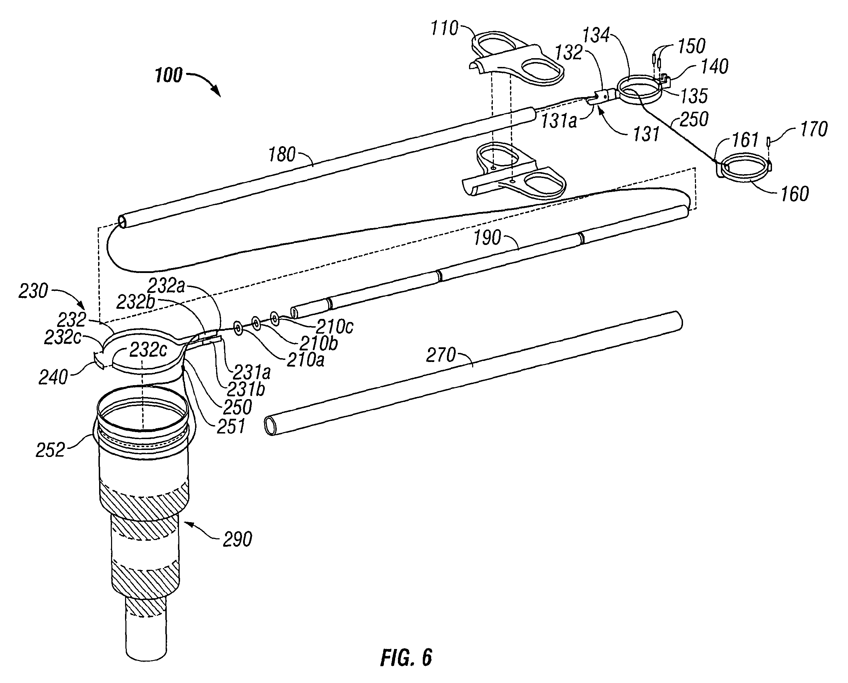

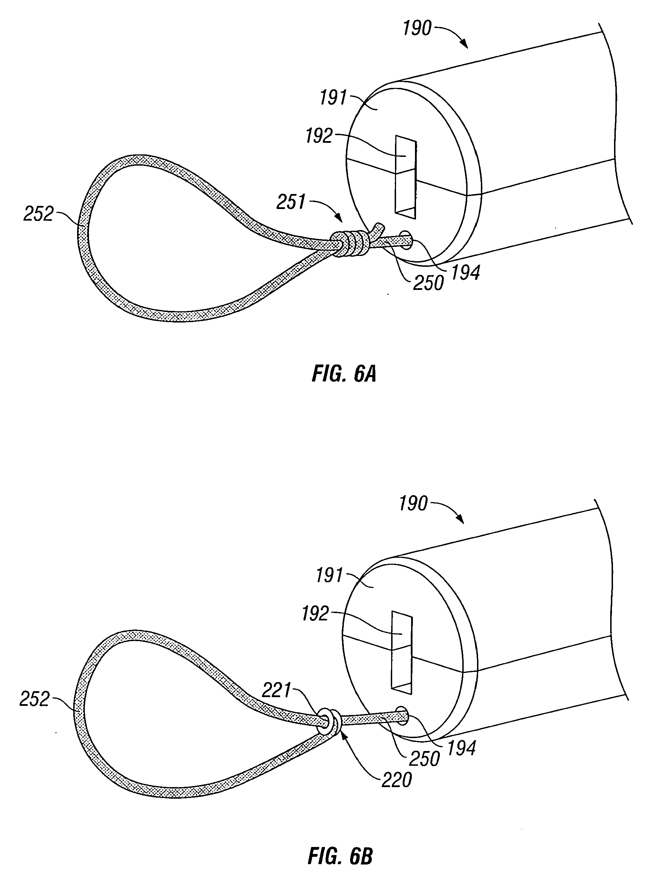

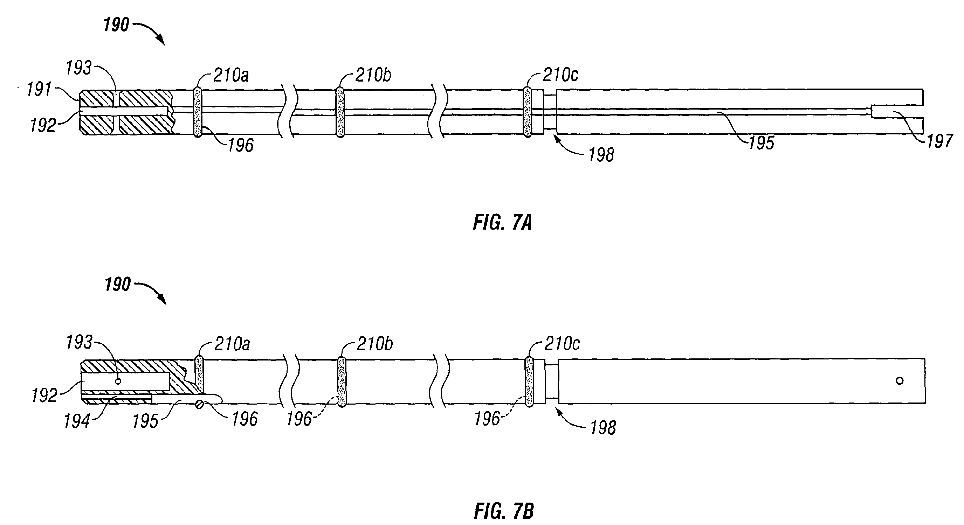

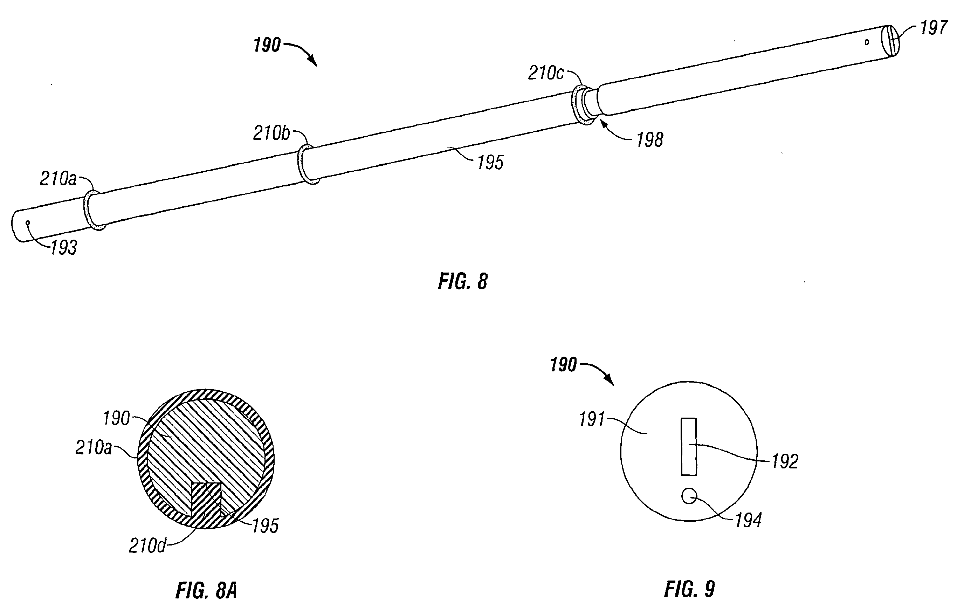

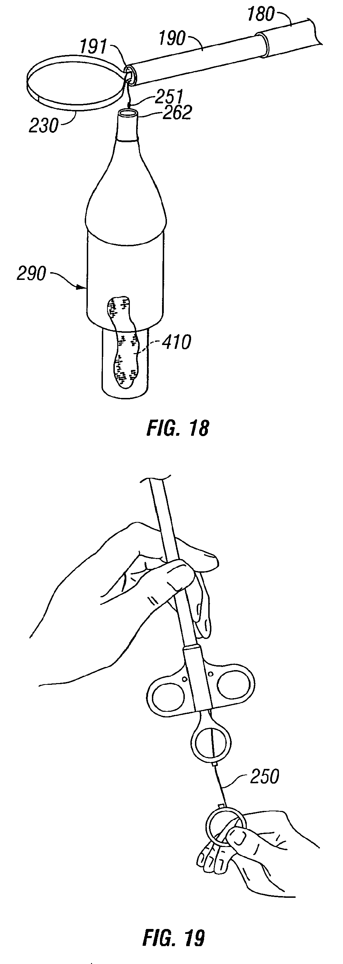

Referring additionally now to FIGS. 7a, 7b, 8, 8a, and 9, the drive rod or bar is an elongated generally cylindrical member slidably disposed through the bore of tube 180. Drive rod 190 includes a distal pushing end 191 and is attached to the pouch assembly to move the pouch from a non-deployed position contained within the outer tube 180 (as shown in FIG. 5) to a deployed position distal to the outer tube 180, (as shown in FIGS. 4a and 18). A spring retainer slot 192 extends longitudinally through the drive rod and opens at the distal end 191. Aperture 193 (FIG. 8) extends transverse to the drive rod 190 across the spring retainer slot 192. Pin 200 is disposed through aperture 193 (FIG. 6) and through apertures 231 b and 232b (See FIG. 6) for fastening spring 230 within retainer slot 192.

Drawstring aperture 194 extends longitudinally through the drive rod opening distally at end 192. Drawstring aperture 194 opens proximally into drawstring slot 195 (see FIG. 7a), which extends longitudinally along the drive rod 190. Drawstring slot 195 terminates at its proximal end at slot 197. Proximal slot 197 is adapted to receive finger loop 130. Drive rod 190 also includes circumferential slots 196 for receiving O-rings 210a, 210b, and 210c. The O-rings help maintain a gaseous seal and/or help to maintain the drawstring in place while permitting sliding movement of the drive rod 190 through tube 180. As shown in FIG. 8a, O-ring 210a includes an inwardly pointing projection 210d for providing additional fluid and gas sealability.

The drive rod 190 is preferably fabricated from a strong polymeric material. A material suitable for fabricating the drive rod 190 is polycarbonate plastic with 20% glass fiber filler. If gamma sterilization is desired, this material has the additional advantage of being gamma stable. Other suitable materials for the purposes discussed herein, as are known in the art, may also be used. To maintain a gaseous seal within the instrument, close tolerances are observed. The outer diameter of the drive rod 190 is slightly less than the inner diameter of the tube 180 through which it slides longitudinally. Additionally, the drive rod 190 is preferably coated with biocompatible lubricant as a viscous sealing material to insure that no gases exit or enter the body through the seal when the operation site (e.g. the peritoneum or other body cavity) is insufflated. Any biocompatible lubricant that will operate as a viscous sealing material may be used, but if gamma sterilization is desired the biocompatible lubricant chosen should be gamma stable. A locking tab 105 (FIG. 5) is included to prevent premature actuation of the instrument during shipping. The locking tab includes snap fit engagement structure to engage slot 198 (FIG. 7b) of the drive rod. When thus engaged, the drive rod cannot be pushed distally beyond the point where the locking tab 105 engages the proximal end of the handle portions 110, 120. To actuate the instrument the surgeon must first disengage the locking tab by pulling it off the instrument.

The spring 230 preferably is formed from two flexible and resilient strips 231 and 232 (FIG. 6) which, in unstressed or freely expanded condition together form a generally circular hoop for supporting the periphery of mouth 264 of pouch assembly 290. Each strip 231 and 232 has a proximal end portion, 231a and 232a, respectively, with apertures 231b and 232b extending laterally therethrough. The proximal end portions 231a and 232a are adapted to be received into slot 192 of the drive rod 190 so that longitudinal movement of the drive rod 190 in the manner described below will move spring 230 and attached pouch assembly 290. Apertures 231 b and 232b are configured so as to align with aperture 193 of the drive rod, thereby permitting disposition therethrough of pin 200. The distal ends 231c and 232c, respectively, meet in opposing relationship where they are joined by tubing 240 made from polymeric, and preferably shrink-wrap type material. In addition, a plurality of arrows or other indicia may be disposed about the tubing 240 to aid in the manufacturing assembly of the instrument and for also helping the surgeon properly orientate the pouch assembly 290, prior to organ entrapment and removal. Spring 230 is preferably fabricated from a resilient metal, such as stainless steel. Other resilient materials are also contemplated, including TINEL brand super elastic metal available from Raychem Corporation of Menlo Park, California and plastic. In other embodiments, the spring 230 may be formed from a unitary resilient strip or section of material.

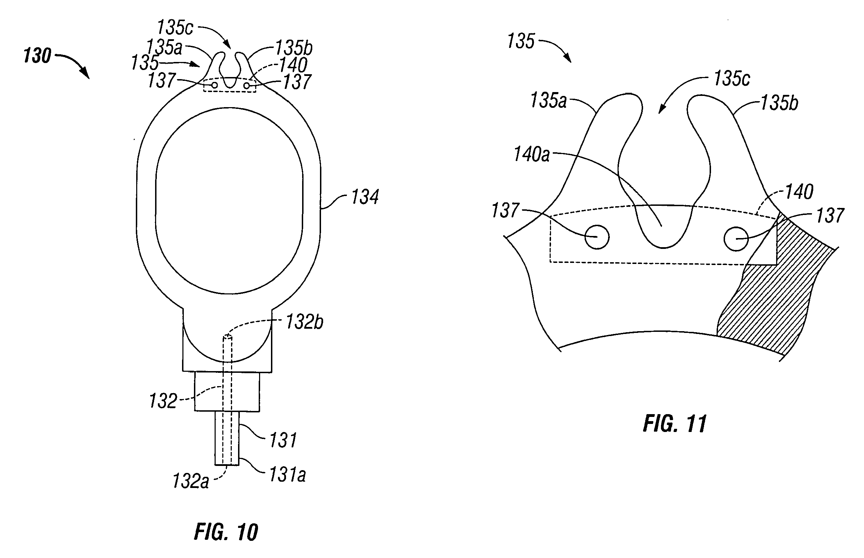

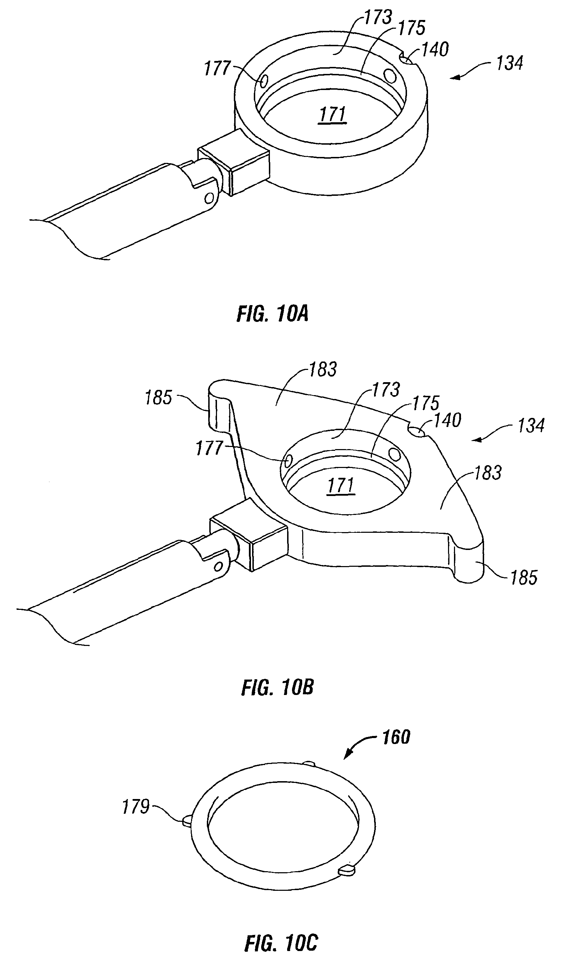

Referring now to FIGS. 6, 10, and 11, the finger loop 130 includes a ring portion 134 for engagement by a user's finger. The distal end of the finger loop includes projection 131 for engaging slot 197 in the drive rod 190, wherein the projection 132 is fixedly attached. Projection 131 includes a key portion 131a which engages a corresponding notch in the handle portion 120 to prevent relative rotation of the drive rod within the apparatus when the pouch is fully deployed. Aperture 132 extends longitudinally from distal opening 132a to proximal opening 132b and it receives thread 250 therein. At its proximal end, finger loop 130 may possess a fixture 135 for releasably holding pull ring 160. Fixture 135 comprises spaced apart generally proximally pointing prongs 135a and 135b which define a proximally opening mouth 135c for snap-fit reception of post 170 of the pull ring 160. In an alternate embodiment shown in FIG. 10a, ring portion 134 on finger loop 130 has an interior recess 171 dimensioned to releasably hold pull ring 160 (FIG. 10c). Interior recess 171 is defined by interior wall 173 of ring portion 134 and shoulder 175. The interior wall 173 contains at least one depression 177, for snap-fit reception of corresponding projections 179 of pull ring 160. In the preferred embodiment, three circumferential depressions 177 are disposed along the interior wall 173, one at a distal end thereof and two opposite each other at a proximal end thereof. As seen in FIG. 10b, ring portion 134 may also include projections 183 to allow the instrument to be gripped with more than one finger, if desired. In the preferred embodiment, projections 183 extend outwardly in a transverse direction from ring portion 160 and include tapered ends 185. Tapered ends 185 may be rounded and may also include a slight bend to facilitate gripping with more than one finger, when desired. A knife 140 is mounted laterally across the mouth at the distal end thereof with sharp knife-edge 140a pointed proximally. The knife 140 is secured at the fixture 135 by pins 150 disposed through apertures 137 in the finger loop 130. The knife-edge 140a functions to cut the drawstring 250, as will be described below. Alternatively, the knife 140 can be insert molded or mounted, onto the finger loop 130.

Referring to FIG. 6, handle portions 110 and 120 are fixedly joined together to form a unitary ring handle, which is fixedly mounted to the proximal end of tube 180 for receipt of a user's finger to facilitate manipulation of the instrument.

Pull ring 160 is a finger ring to facilitate pulling of drawstring 250 to which it is attached, preferably using an adhesive. In one embodiment, post 170, which may be in integral part of pull ring 160, is pivotally mounted into mouth 135c of fixture 135 (FIG. 10) in a snap-fit engagement and may be disengaged by exerting a pulling force thereon to separate ring 160 from ring portion 134 for reasons discussed below. When utilizing post 170, pull ring 160 preferably includes projection 161 which is engageable with a corresponding depression in finger loop 130 to prevent inadvertent or unintended pivoting of the pull ring 160. In an alternate embodiment, FIG. 10b, pull ring 160 includes at least one projection 179 for snap-fit engagement with corresponding depressions 177 of ring portion 134 and may be disengaged by exerting a pulling or pushing force sufficient to separate pull ring 160 from ring portion 134 for reasons discussed below. In a preferred embodiment, the pull ring is distinctively colored to alert the user as to the orientation of the pouch assembly 290.

Drawstring 250 is tied at one end to pull ring 160, and extends through aperture 132 in the finger loop, through drawstring slot 195 in the drive rod 190, through drawstring aperture 194, and around the mouth 264 of the first sac 260 by passing through lower tubular chamber 266 (see FIG. 6). The drawstring 250 is preferably coated with a biocompatible lubricant as a viscous sealing material to insure that gases do not enter or exit the peritoneum through aperture 194. Any biocompatible lubricant that will operate as a viscous sealing material may be used, but if gamma sterilization is desired the biocompatible lubricant chosen should be gamma stable. The end of the drawstring 250 is brought around and tied to the drawstring 250 by knot 251 to form loop 252. Knot 251 is a "running knot," i.e. a knot that slips along the rope or line around which it is tied. Thus, loop 252 tightens when the standing part of the line is pulled. For proper operation, knot 251 should have a size larger than the diameter of aperture 194 (FIGS. 6a and 6b). When drawstring 250 is pulled proximally, the knot 251 will be pulled up to the distal opening of aperture 194 where the knot 251 will abut the distal face 191 of drive rod 190. Aperture 194 has a diameter large enough to admit a single strand or filament of drawstring 250 with minimal clearance to help maintain a gaseous seal with further sealing provided by the biocompatible lubricant, but not large enough to permit knot 251 to pass therethrough. Thus, knot 251 is retained in position while drawstring 250 is pulled proximally, thereby closing loop 252. This, in turn, closes mouth 262 of the first sac 260 and detaches pouch assembly 290 along scored line 265. Use of the running knot enables closing and opening of the pouch to be achieved by a single actuating filament of drawstring 250 moving through the apparatus. An aperture or thread passage in a laparoscopic instrument for accommodating a single filament of thread need not have as large a diameter as that for accommodating two or more filament of drawstring thread.

A further advantage is that it is easier to maintain a proper gaseous seal within aperture 194 when a single filament is moved therethrough than if two or more filaments of thread were disposed therethrough. Although apparatus configurations having only a single actuating filament are preferred, also contemplated as being within the scope of the present disclosure are apparatus employing multiple actuating filaments from, for example, two or more filaments, or doubled-over single filaments.

Any type of running knot having the proper diameter may be used, such as the slipknot or running bowline, and variations thereof. The knot preferably should maintain enough friction on the drawstring such that the knot slides along the drawstring when the drawstring is pulled with sufficient tension. The knot should also preferably slide in both a distal and proximal direction for closing, and if necessary to facilitate removal of tissue, opening of the pouch after detachment.

The present disclosure contemplates structures and/or devices other than knots for accomplishing the same function as described above. For example, rings, eyelets, and the like may be used. As shown in FIG. 6b, one end of the drawstring filament is attached to ring member 220, which has an aperture 221 for receiving drawstring 250. When the drawstring 250 is pulled proximally, ring member 220 abuts the proximal face 181 at aperture 194, permitting drawstring 250 to be pulled through, thereby closing loop 252.

Thus, any structure or device for slidably attaching one end of the drawstring to the drawstring thread to form a reducible loop, or running noose, is contemplated as being within the scope of the present disclosure.

Tube 180 is of such diameter as to permit it to be slidably disposed through a trocar cannula for use in endoscopic or laparoscopic operations, and is generally between about 0.25 inches to about 0.50 inches in diameter, and about 10 inches to about 15 inches long, although other dimensions may also be used if appropriate to the operation being performed. Tube 180 slidably houses the drive rod 190 and, when undeployed, the spring 230 and pouch assembly 290. In the initial, unused condition, pouch assembly 290 will be rolled up and spring portions 231 and 232 will be relatively straight and positioned within tube 180. When the drive rod 190 is advanced, the spring 230 connected thereto will exit the distal end of tube 180 and resiliently pop open, thereby deploying and opening pouch assembly 290. Tube 180 is preferably formed from a metal such as stainless steel and is preferably coated with a shrink-wrap plastic such as shrinkable polyethylene fiberglass, or polyvinyl chloride of a grade suitable for use in surgical procedures.

Minimally invasive surgery in the abdomen usually requires the placement of one or more trocar assemblies in the abdominal wall to provide access to the peritoneum for the surgical instruments. The trocar assembly may include an obturator with a sharp, tissue piercing point, a cannula having a tube and a proximal section which usually includes valve and sealing structures. The surgeon inserts the trocar assembly into the abdominal wall and then removes the obturator leaving the cannula inserted into the body cavity and the proximal section outside the body. The body cavity is then insufflated. Additional cannulae can be inserted and various operating and optical viewing instruments may be inserted through the several cannulae. The cannula sealing structure helps prevent the entry or escape of gas between the inside of the cannula and the outside of the instrument. As mentioned before, the instruments generally have internal sealing structure to prevent the escape or entry of gas through the interior of the instrument. Placement of trocar cannulae and insertion of instruments therethrough are performed in accordance with methods and apparatus known and commonly available to those of skill in the art.

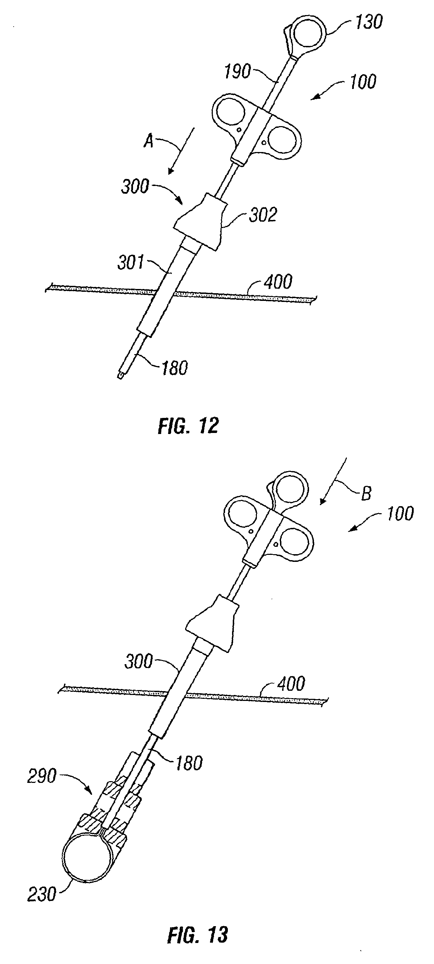

Referring now to FIGS. 12 to 22, a method of using the apparatus of the present disclosure in minimally invasive surgery will now be described. By way of illustration, surgical procedures in which the method of the present disclosure may be used include, but are not limited to, nephrectomy, cholecystectomy, appendectomy, and the like.

FIG. 12 shows a diagrammatic view of a first trocar cannula 300 inserted through a wall of body tissue 400 to gain access to a body cavity, such as for example the peritoneum. The applicator assembly 100, with the specimen retrieval pouch assembly 290 in the non-deployed position is inserted through the cannula 300 in the direction of arrow A such that the distal end of the applicator assembly 100 is positioned within the body cavity. As depicted in FIG. 12, the applicator assembly is in the initial condition with specimen retrieval pouch assembly 290 retained within tube 180. Pull ring 160 is positioned atop post 170. The locking tab 105 is removed at this time to permit actuation of the instrument.

Referring now to FIG. 13, the drive rod 190 is advanced longitudinally distally by the surgeon's pushing of the finger loop 130 as indicated by arrow B. The finger loops of ring portion 110 (and 120) may be grasped by the user during the movement of finger loop 130. This movement, of drive rod 190 advances the pouch assembly 290 beyond the distal end of tube 180 where spring 230 is no longer restrained by tube 180 and, therefore, resiliently pops open to its substantially round configuration to thereby move the mouth 262 of the first sac 260 from its closed configuration to its open configuration.

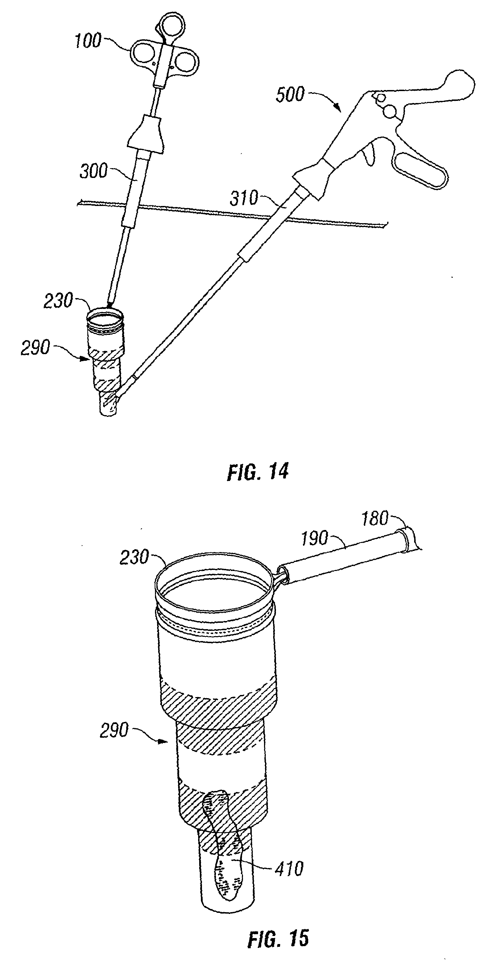

Referring now to FIG. 14, non-traumatic forceps or graspers 500 may be inserted through a second trocar cannula 310 and manipulated for gently unrolling the pouch assembly 290 if necessary.

Referring to FIG. 15, a specimen of body tissue 410 is excised and placed into pouch assembly 290. The specimen 410 may optionally be treated, i.e. morcellated or otherwise divided prior to removal from the body cavity.

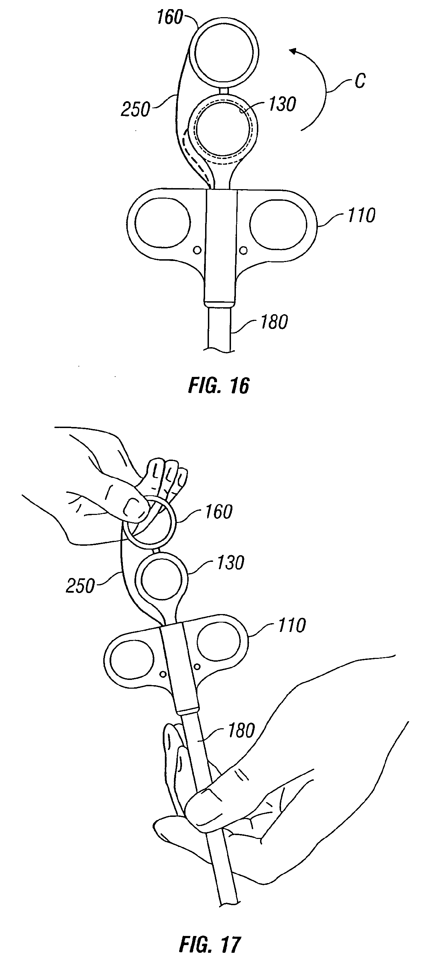

Referring to FIG. 16, the pull ring 160 is pivoted with post 170 in a direction as shown by arrow C to a position as illustrated in FIG. 17.

Referring to FIG. 17, the pull ring 160 is grasped and pulled thereby disengaging post 170 from fixture 135 and permitting removal of pull ring 160 from the finger loop 130.

When the pull ring 160 is pulled (FIG. 18), drawstring 250 is moved proximally, thereby detaching the pouch assembly 290 from the spring support 230 along scored line 265. Continued pulling of the drawstring 250 will bring running knot 251 into abutment with the distal end 191 of the pusher thus reducing noose or loop 252 and closing the mouth 262 of the pouch assembly 290.

Referring to FIG. 19, the drawstring 250 is inserted into mouth 135c of fixture 135 and cut by knife 140 to allow for subsequent removal of the instrument with the closed pouch remaining inside the body cavity.

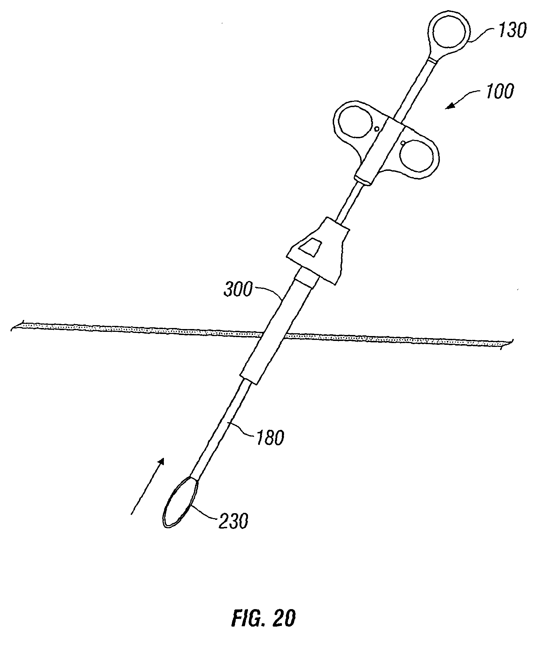

Referring to FIG. 20 the finger loop 130 is pulled to withdraw spring 230 back into tube 180, whereupon spring 230 refolds back into its pre-deployed relatively straight configuration to permit removal of the apparatus 100 from the cannula 300. The drive rod 190 is not retracted completely out of the proximal end of tube 180, since complete retraction of the drive rod will permit exit of the spring 230 and subsequent opening of the spring 230 outside the proximal end of the apparatus.

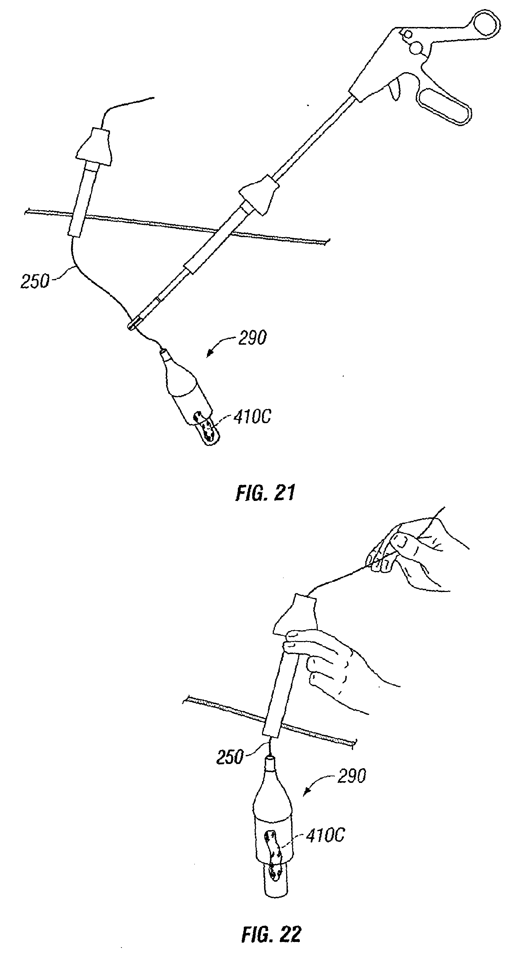

At this point drawstring 250 is sitting in the cannula and as shown in FIG. 22, the pouch assembly 290 with tissue specimen 410 can be immediately removed through the trocar site by pulling the drawstring 250 through the cannula until the end of the pouch assembly 290 reaches the neck of the trocar and both can be removed together, or the trocar can be removed first and removed thereafter through the same incision. By advantageously using a number of differently dimensioned sacs to form the pouch assembly 290, agglomeration of the tissue sample 410 is minimized thereby facilitating the retrieval of the pouch assembly 290 that contains the tissue specimen 410. Further still, the tissue specimen 410 will tend to conform to the "sausage" shape of the staggered sacs in pouch assembly 290 thereby further facilitating the retrieval of the pouch assembly 290 with its contents.

Alternatively, with the drawstring securely holding the mouth 262 of the pouch assembly 290 closed, the drawstring 250 may be grasped by an appropriate endoscopic instrument, such as a grasper, held inside the body cavity, as shown in FIG. 21, and removed later during the operation. If necessary, the incision may be enlarged to permit passage therethrough of the pouch assembly 290 and specimen 410. However, it is alternatively contemplated that if the specimens 410 contained in the pouch assembly 290 is sufficiently small, or if divided as discussed above, it can be removed through the cannula.

While the above description contains many specifics, these specifics should not be construed as limitations on the scope of the present disclosure, but merely as exemplifications of preferred embodiments thereof. Those skilled in the art will envision many other possible variations that are within the scope and spirit of the present disclosure.

## Claims

1. A surgical apparatus for removing tissue from an interior portion of a body during a surgical procedure comprising:
a pouch assembly including a support and a pouch, said pouch including at least a first sac having a second diameter, the first sac having an open distal end in fluid communication with an open proximal end of the second sac, the first diameter being greater than the second diameter and the second sac having a closed distal end, the pouch being dimensioned and arranged for receiving the tissue;
a drawstring operatively coupled to a proximal region of the first sac; and
an elongate tubular member for introducing the pouch, the pouch assembly being attached to the elongate tubular member.

2. The surgical apparatus of claim 1, wherein the first sac proximal end has a proximal sleeve for receiving a spring member supported by the elongate tubular member.

3. The surgical apparatus of claim 2, wherein the first sac has a distal sleeve disposed distally of the proximal sleeve.

4. The surgical apparatus of claim 3, further comprising a drawstring disposed in the distal sleeve.

5. The surgical apparatus of any one of the preceding claims, wherein the first sac defines a weakened portion adjacent the support.

6. The surgical apparatus of any one of the preceding claims, wherein the pouch is formed from a translucent material.

7. The surgical apparatus of any one of the preceding claims, wherein the firs sac has a substantially uniform diameter.

8. The surgical apparatus of any one of claims 1 to 6, wherein the first sac has a tapered configuration.

9. The surgical apparatus of any one of the preceding claims, wherein the second sac has a substantially uniform diameter.

10. The surgical apparatus of any one of claims 1 to 8, wherein the second sac has a tapered configuration.

11. The surgical apparatus of any one of the preceding claims, wherein the pouch has a reinforced band overlapping the first sac and the second sac.

12. The surgical apparatus of any one of the preceding claims, wherein the elongate tubular member has a drive rod disposed therein, the pouch assembly being operably attached to the drive rod.

13. The surgical apparatus of claim 12, wherein the tubular member is sized so that the pouch assembly is received therein and the support is attached to the drive rod so that movement of the drive rod from a first position in which the pouch assembly is disposed within the tubular member to a second position in which the pouch assembly exits the tubular members.

14. The surgical apparatus of any one of the preceding claims, wherein the drawstring is received in the tubular member and attached to a handle for pulling the drawstring proximally and thereby closing the pouch.

15. The surgical apparatus of claim 14, wherein the drive rod is attached to a handle for pulling the handle and moving the drive rod from the first position to the second position.
